# EUROPEAN PATENT SPECIFICATION

(11) **EP 1 842 908 B1**
(45) Date of publication and mention of the grant of the patent: **17.11.2010**
(21) Application number: 06711875.2
(22) Date of filing: 18.01.2006
(51) Int. Cl.: C12N 9/20, C12P 7/64

(54) **PROCESS FOR PRODUCTION OF PURIFIED LIPASE**
VERFAHREN ZUR HERSTELLUNG VON GEREINIGTER LIPASE
PROCÉDÉ DE PURIFICATION DE LIPASE

(30) Priority: 19.01.2005 JP 2005011770; 19.01.2005 JP 2005011771
(43) Date of publication of application: 10.10.2007
(60) Divisional application: 09004164.1
(73) Proprietor: The Nisshin OilliO Group, Ltd., Tokyo 104-8285 (JP)
(72) Inventor: NEGISHI, Satoshi, The Nisshin OilliO Group, Ltd, Yokosuka-shi, Kanagawa 239-0832 (JP); SUZUKI, Junko, The Nisshin OilliO Group, Ltd., Yokosuka-shi, Kanagawa, 239-0832 (JP); SAKURAI, Chika, The Nisshin OilliO Group, Ltd., Yokosuka-shi, Kanagawa, 239-0832 (JP); ARIMOTO, Shin, The Nisshin OilliO Group, Ltd., Yokosuka-shi, Kanagawa, 239-0832 (JP); UEHARA, Hidetaka, The Nisshin OilliO Group, Ltd., Yokosuka-shi, Kanagawa, 239-0832 (JP); HIROSE, Tadashiro, The Nisshin OilliO Group, Ltd., Yokosuka-shi Kanagawa, 239-0832 (JP); YAMAUCHI, Yoshie, The Nisshin OilliO Group, Ltd., Yokosuka-shi, Kanagawa, 239-0832 (JP); ARAI, Yuri, The Nisshin OilliO Group, Ltd., Yokosuka-shi, Kanagawa, 239-0832 (JP); SUGANUMA, Tomomi, The Nisshin OilliO Group, Ltd., Yokosuka-shi, Kanagawa, 239-0832 (JP)
(74) Representative: Bawden, Peter Charles
(86) International application number: PCT/JP2006/300606
(87) International publication number: WO 2006/077860

(56) References cited:
- EP-A- 1 795 591
- WO-A-90/04033
- WO-A-90/12858
- WO-A-02/081719
- JP-A- 08 089 265
- MUSTRANTA A ET AL: "Applications of immobilized lipases to transesterification and esterification reactions in nonaqueous systems" ENZYME AND MICROBIAL TECHNOLOGY, STONEHAM, MA, US, vol. 15, no. 2, 1 January 1993 (1993-01-01), pages 133-139, XP002456966 ISSN: 0141-0229
- NEGISHI SATOSHI ET AL: "Activation of powdered lipase by cluster water and the use of lipase powders for commercial esterification of food oils" ENZYME AND MICROBIAL TECHNOLOGY, STONEHAM, MA, US, vol. 32, no. 1, 2 January 2003 (2003-01-02), pages 66-70, XP002456965 ISSN: 0141-0229
- MOGI K. ET AL.: 'Transesterification reaction between medium- and long-chain fatty acid triglycerides using surfactant-modified lipase' BIOTECH. BIOENG. vol. 67, no. 5, 2000, pages 513 - 519, XP003004057

## Description

### Technical Field of the Invention

The present invention relates to a method for producing a purified lipase which can be suitably used in various esterification reactions or ester exchange reactions, a method for producing a lipase for ester exchanges which can be suitably used in the ester exchange reactions, a method for exchanging esters of fat and fatty oil with the lipase obtained by the above methods, and a lipase composition containing the lipase.

### Background of the Invention

Lipases are widely used in esterification reactions of various carboxylic acids such as a fatty acid with alcohols such as monoalcohol and polyalcohol or ester exchange reactions between several carboxylic esters. Among them, the ester exchange reactions are important technology including modification of fat and fatty oil of animals and plants and as a method for producing esters of various fatty acids, sugar esters or steroids. When lipases that are fat and fatty oil hydrolases are used as a catalyst in those reactions, ester exchange reactions can be conducted under the temperate condition of room temperature to about 70°C. Therefore, use of lipases can not only inhibit side reactions and reduce energy costs as compared with the prior chemical reactions, but also its safety is high because the lipases as the catalyst are natural products. Further, objective compounds can be effectively produced due to its substrate specificity or positional specificity.

On the other hand, since silicon is added into a usual medium culture for defoaming during culture of producing microorganism of lipases, the added silicon is mixed in the collected lipases. When ester exchange of fat and fatty oil is conducted using such lipases, silicon is sometimes brought into the obtained ester-exchanged fat and fatty oil, and therefore, it causes a problem that foaming increases when food is fried with oil containing silicon. Meanwhile, during culture of producing microorganism of lipases, the lipases sometimes generate pigments. When ester exchange of fat and fatty oil is conducted using such lipases, the obtained ester-exchanged fat and fatty oil sometimes is stained or their flavor deteriorates.

Thus, purified lipases have been desired wherein various impurities that are supposed to be derived from silicon or lipase are highly removed.

In addition, lipases for ester exchanges have been desired wherein lipase activity is improved. Mogi K et A1 in Biotech Bioeng Vol 67 no 5 2000 pages 513 - 519 describe transesterification reaction between medium - and long - chain fatty acid triglycerides using surfactant - modified lipose.

### Disclosure of the Invention

The object of the present invention is to provide a method for producing a (purified) lipase.

The further object of the present invention is to provide a method for producing a lipase (for ester exchanges).

The other object of the present invention is to provide a method for producing fat and fatty oil compositions using the lipase obtained by the above production method.

The object of the present invention is also to provide a lipase composition containing the lipase obtained by the above production method.

The present invention has been completed on the basis of the finding that lipases can be purified by mixing them with a long chain fatty acid triglyceride and a medium-chain triglyceride to effectively migrate various impurities in the lipases to the triglyceride.

Namely, the present invention provides a method for producing a (purified) lipase which comprises steps of:
(a) bringing a long chain fatty acid triglyceride and a medium-chain triglyceride into contact with a lipase by stirring a mixture of said triclycerides, lipase and a filter aid for 0.5 to 100 hours at ambient temperature to purify the lipase wherein 2 to 100 times mass of triclycerides is brought into contact with a total mass of the lipase; and
(b) collecting the purified lipase by filtration

The present invention further provides a method for producing a fat and fatty oil composition which comprises a step of exchanging esters of the fat and fatty oil in the presence of the lipase produced by the above production method.

The present invention additionally provides a lipase composition containing the lipase produced by the above methods and 0.5 to 5 times mass of triglycerides to that of the lipase.

### Best Mode for Carrying out the Invention

### [Long chain fatty acid triglycerides]

Long chain fatty acid triglycerides used in the present invention are preferably a triglyceride having 14 to 24 and more preferably 16 to 18 carbon atoms in a constituent fatty acid. The constituent fatty acid may be saturated or unsaturated, and it may be a linear chain or a branched chain. Three fatty acids which constitute triglycerides may be all the same; two of the constituent fatty acids may be the same and the rest may be different from the other two; or three of them may be all different from each other. An unsaturated fatty acid having 16 to 22 carbon atoms is preferable, and a linear unsaturated fatty acid having 16 to 18 carbon atoms is especially preferable. It is particularly preferable to be selected from the group consisting of canola oil, soybean oil, sunflower oil, safflower oil and corn oil. These long chain fatty acid triglycerides can be used on its own, or two or more of them can be used at the same time.

Long chain fatty acid triglycerides which can be used in the present invention can be produced by publicly known methods or commercially available products thereof can be used. As for the commercially available products, for example, it is marketed as a trade name: Canola Oil produced by The Nisshin OilliO Group, Ltd.

### [Medium-chain triglycerides]

Medium-chain triglycerides used in the present invention are preferably a triglyceride having 6 to 12 and more preferably 8 to 10 carbon atoms in a constituent fatty acid. The constituent fatty acid may be saturated or unsaturated, and it may be a linear chain or a branched chain. Three fatty acids which constitute triglycerides may be all the same; two of the constituent fatty acids may be the same and the rest may be different from the other two; or three of them may be all different from each other. A saturated fatty acid having 8 to 10 carbon atoms is preferable. These medium-chain triglycerides can be used on its own, or two or more of them can be used at the same time.

Medium-chain triglycerides which can be used in the present invention can be produced by publicly known methods or commercially available products thereof can be used. As for the commercially available products, for example, it is marketed as a trade name: ODO produced by The Nisshin OilliO Group, Ltd.

In case that the purified lipase of the present invention is used as a catalyst for ester exchanges of fat and fatty oil, a long chain fatty acid triglyceride and/or a medium-chain triglyceride used in the present invention are preferably the same as the fat and fatty oil which will exchange esters.

In the present invention, the long chain fatty acid triglyceride and the medium-chain triglyceride are preferably used in the mass ratio of 99:1 to 1:99, and more preferably in the ratio of 95:5 to 50:50. It is further more preferable that they are used in the ratio of 90:10 to 80 to 20, because the use in such ratios improves operability.

In the present invention, 2 to 100 times mass and more preferably 5 to 10 times mass of triglyceride is brought into contact with a total mass of lipase, because the use in such amounts enhance the cleansing effect of enzymes.

### [Lipases]

Lipases which can be used in the present invention include lipoprotein lipase, monoacylglycerolipase, diacylglycero-lipase, triacylglycero-lipase, galactolipase, and phospholipase. Among them, triacylglycero-lipase is preferable.

Microorganism which generate those lipases are not limited to bacteria, yeast, filamentous bacteria, streptomyces and the like, they include *Pseudomonas* sp., *Alcaligenes* sp., *Arthrobacter* sp., *Staphylococcus* sp., *Torulopsis* sp., *Escherichia* sp., *Mycotorula* sp., *Propionibacterum* sp., *Chromobacterum* sp., *Xanthomonas* sp., *Lactobacillus* sp., *Clostridium* sp., *Candida* sp., *Geotrichum* sp., *Sacchromycopsis* sp., *Nocardia* sp., *Fuzarium* sp., *Aspergillus* sp., *Penicillium* sp., *Rhizomucor* sp., *Mucor* sp., *Rhizopus* sp., *Phycomyces* sp., *Puccinia* sp., *Bacillus* sp., and *Streptmyces* sp.

In the present invention, lipases originated from Alcaligenes sp., *Pseudomonas* sp., *Rhizomucor* sp., *Mucor* sp., *Thermomyces* sp., *Rhizopus* sp., or *Penicillium* sp. are preferable. Further, lipases originated from *Rhizomucor* miehei and *Alcaligenes* sp. are preferable.

Lipases used in the present invention may have or not have a positional specificity. In case that the lipases have the positional specificity, they are preferably 1,3-specific.

Lipases used in the present invention may be in the form immobilized to carriers such as anion-exchange resins, phenol absorbing resins, hydrophobic carriers, cation-exchange resins, and chelate resins, or in the powdery form which is not immobilized to carriers. The powdery form which is not immobilized to carriers is preferable. Such powdery form (hereinafter referred to as powder lipases) is preferable because a purified lipase obtained by the production method of the present invention has much higher enzymatic activity.

When the lipases are powder lipases, particle size thereof can be optional, but 90 weight% or more thereof are 1 to 100 µm and preferably 10 to 70 µm. It is preferable that the size is within such range because the operability is high. Meanwhile, the particle size of powder lipases can be determined by using, for example, a particle size distribution analyzer (LA-500) of HORIBA, Ltd.

Further, it is preferable that water content of a lipase is adjusted to 0.1 to 10% before or after the reaction. It is preferable that the content is within such range because the operability is high.

As for lipases used in the present invention, commercially available ones can be used. Such lipases are, for example, marketed as trade names of Lipase QLM or Lipase PL from Meito Sangyo Co., Ltd., and Lipase RM or Lipase TL from NOVOZYMES.

In case that the lipases used in the present invention are powdery, such lipases can be obtained, for example, by spray-drying or freeze-drying an aqueous solution(s) containing a lipase.

Such aqueous solutions containing the lipase include lipase culture from which fungus bodies are removed, purified culture, those that lipase powder obtained from the above is dissolved/dispersed into water again, those that commercially available lipase powder is dissolved/dispersed into water again, and commercially available liquid lipase. Further, it is more preferable to remove low-molecular components such as salts in order to enhance more the lipase activity, and it is more preferable to remove low-molecular components such as sugar in order to enhance more the powder property.

As for lipase culture, for example, it includes soybean flour, peptone, corn-steep liquor, aqueous solutions containing K₂HPO₄, (NH₄)₂SO₄, MgSO₄ · 7H₂O and the like. The concentration thereof is 0.1 to 20 weight% soybean flour and preferably 1.0 to 10 weight%; 0.1 to 30 weight% peptone and preferably 0.5 to 10 weight%; and 0.01 to 20 weight% K₂HPO₄ and preferably 0.1 to 5 weight%. Further, (NH₄)₂SO₄ is preferably 0.01 to 20 weight% and preferably 0.05 to 5 weight%; and MgSO₄ · 7H₂O is preferably 0.01 to 20 weight% and preferably 0.05 to 5 weight%. The cultural conditions are cultivation temperature of 10 to 40°C and preferably 20 to 35°C; draft quantity of 0.1 to 2.0VVM and preferably 0.1 to 1.5VVM; rotation speed of stirring of 100 to 800 rpm and preferably 200 to 400 rpm; and pH of 3.0 to 10.0 and preferably 4.0 to 9.5.

Separation of fungus bodies is preferably conducted by centrifugation, membrane filtration, and the like. Removal of the low-molecular components such as salts and sugar can be conducted by ultrafiltration treatment. Concretely, an aqueous solution containing a lipase from which low-molecular components are removed can be obtained by treating ultrafiltration to condense an aqueous solution containing a lipase to its 1/2 volume, and then by adding once to five times the same amount of a phosphoric buffer as that of the condensed solution.

It is preferable that centrifugation is 200 to 20,000 × g; and in membrane filtration, the pressure is controlled to 3.0kg/m2 or lower by microfiltration or a filter press. In case of enzymes in fungus bodies, it is preferable that they are cell-ground by a homogenizer, a Waring blender, ultrasonic disintegration, a French press, a ball mill and the like; and residue of cells is removed by centrifugation, membrane filtration and the like. The rotation speed of stirring of a homogenizer is 500 to 30,000rpm and preferably 1,000 to 15,000 rpm; and revolution of a Waring blender is 500 to 10,000 rpm and preferably 1,000 to 5,000rpm. The time of stirring is 0.5 to 10 minutes and preferably 1 to 5 minutes. The ultrasonic disintegration is conducted under the condition of 1 to 50KHz and preferably 10 to 20KHz. In the ball mill, it is preferable to use a glass pellet having a diameter of around 0.1 to 0.5mm.

In the present invention, it is preferable to use an aqueous solution containing a lipase such as those containing 5 to 30 weight% solid contents of the lipase.

It is preferable that pH of the aqueous solution containing a lipase is adjusted to 6 to 7.5 right before the drying process such as spray-drying. It is particularly preferable that pH thereof is adjusted to 7.0 or lower and more preferably to 6.5 to 7.0. The adjustment of pH may be conducted in any processes before the drying process such as spray-drying. The pH of the aqueous solution containing a lipase may be preliminarily adjusted so that the pH right before the drying process becomes within the above range. Various alkali agents or acids can be used to adjust pH, and it is preferable to use alkali metal hydroxides such as sodium hydroxide.

Further, in some stage before the drying process, the aqueous solution containing a lipase may be concentrated. The concentration methods are not particularly limited, and they include an evaporator, a flash evaporator, concentration by ultrafiltration, concentration by microfiltration, salting out by inorganic salts, precipitation by solvents, absorption methods by ion-exchange cellulose and the like, and water absorption methods by water-absorbing gel. The concentration by ultrafiltration and an evaporator are preferable. The module for the concentration by ultrafiltration is a flat membrane or a hollow fiber membrane having a molecular weight cut off of 3,000 to 100,000 and preferably 6,000 to 50,000. The materials thereof are polyacrylnitrile, polysulfone and the like.

It is preferable that spray-drying is conducted, for example, by spray dryers such as nozzle countercurrent flow, disk countercurrent flow, nozzle concurrent flow and disk concurrent flow, and the disk concurrent flow is more preferable. The spray-drying is preferably controlled as follows: the rotation speed of the atomizer is 4,000 to 20,000rpm; and heating is 100 to 200°C for inlet temperature and 40 to 100°C for outlet temperature.

Further, freeze-drying is also preferable. For example, it is preferable that freeze-drying is conducted by a laboratory-size small freeze dryer or plate type freeze-drying. It can also be prepared by drying under reduced pressure.

In the process (a) of the present invention, a lipase is purified by bringing a long chain fatty acid triglyceride and a medium-chain triglyceride into contact with the lipase. The contact can be conducted, for example, by adding the mixture of a long chain fatty acid triglyceride and a medium-chain triglyceride to a lipase, and then by stirring the mixture for 0.5 to 100 hours at ambient temperature. The purified lipase having a high purity can be obtained by stirring.

After the process (a), the lipase is taken from the triglyceride by filtration and the like, and so in the process (a), the mixture is brought into contact with a filter aid(s) because it enhances the operability. As for an example of the filter aids which can be used in the present invention, there is a trade name: KC Flock produced by Nippon Paper Chemicals Co., Ltd.

In the process (a), a partial ester of a fatty acid of glycerin and/or a partial ester of a fatty acid of glycerin condensation may be brought into contact with a lipase in addition to triglyceride. It is preferable to bring a partial ester of a fatty acid of glycerin and/or a partial ester of a fatty acid of glycerin condensation into contact with a lipase because the color and the smell of the obtained fat and fatty oil compositions are good.

### [Partial esters of a fatty acid of glycerin and partial esters of a fatty acid of glycerin condensation]

Partial esters of a fatty acid of glycerin include monoglyceride, diglyceride and the like. As for monoglyceride which can be used, there are esters of fatty acid(s) having 8 to 22 carbon atoms and glycerin. Concretely, they include oleic acid monoglyceride, linoleic acid monoglyceride, stearic acid monoglyceride, and palmitic acid monoglyceride, and oleic acid monoglyceride is preferable. Two or more monoglycerides may be used together. As for diglyceride, there are esters of fatty acid(s) having 8 to 18 carbon atoms and glycerin. Concretely, they include oleic acid diglyceride, linoleic acid diglyceride, stearic acid diglyceride, and palmitic acid diglyceride, and oleic acid diglyceride is preferable. Two or more diglycerides may be used together.

(g1) A partial ester of a fatty acid of glycerin and/or a partial ester of a fatty acid of glycerin condensation and (g2) triglyceride are preferably used in the mass ratio (g1)/(g2) of 1:99 to 99:1. They are more preferably used in the ratio of 1:99 to 60:40. (g1) Monoglyceride and/or diglyceride and (g2) triglyceride are preferably used in the mass ratio (g1)/(g2) of 0.1:99.9 to 99:1. They are more preferably used in the ratio of 0.1:99.1 to 90:10 because ester exchanged oil having a good color can be obtained.

In case of using a partial ester of a fatty acid of glycerin in combination with a partial ester of a fatty acid of glycerin condensation, the partial ester of a fatty acid of glycerin and the partial ester of a fatty acid of glycerin condensation are preferably used in the mass ratio of 99:1 to 1:99. They are more preferably used in the ratio of 99:1 to 50:50. In case of using a monoglyceride in combination with a diglyceride, they are preferably used in the ratio of 1:99 to 90:10 because the use in such a ratio enhances the operability.

Partial esters of a fatty acid of glycerin condensation include esters with a fatty acid having 8 to 18 carbon atoms. Concretely, they include an ester of oleic acid polyglycerin. Among them, an ester of oleic acid decaglycerin is preferable.

Partial esters of fatty acids of two or more glycerin condensations may be used together.

A partial ester of a fatty acid of glycerin and/or a partial ester of a fatty acid of glycerin condensation can be produced by publicly known methods such as that liquid fat and fatty oil like canola oil is hydrolyzed and then molecular-distilled. Commercially available products are also usable. For example, as for monoglyceride, a trade name: Sunsoft 8090 produced by Taiyo Kagaku Co., Ltd. can be used.

In the process (a), it is preferable from the viewpoint of repeated reactions that a filter aid(s) is used in combination with a partial ester of a fatty acid of glycerin and/or a partial ester of a fatty acid of glycerin condensation.

The purified lipase thus produced can be used as itself. However, it is preferable, from the point of tractability, that it is used as a lipase composition wherein the lipase is dipped or infiltrated in triglyceride. Here, the mass of the triglyceride in the lipase composition is preferably 0.5 to 5 times and more preferably 1 to 3 times as heavy as that of the lipase.

The lipase obtained from the present invention can be suitably used as a lipase for ester exchanges. The ester exchange reactions of triglyceride and the like can be effectively conducted with the above lipase in accordance with the known method. For details, the lipase can be used in the reaction of ester and alcohol or a carboxylic acid in addition to the reaction between esters. Further, the lipase of the present invention can also be used in the reaction synthesizing esters from alcohol and a carboxylic acid.

More specifically, ester exchanges are conducted by reacting a compound(s) having at least one alcoholic hydroxyl group(s) in the molecular, a carboxylic acid or its ester to an ester. Examples of the esters which are subjects of ester exchanges include triglycerides having 4 to 22 carbon atoms. Specifically, they include canola oil, soybean oil, sunflower oil, corn oil, rice bran oil, safflower oil, sesame oil, linseed oil, palm oil, and fractionated oils thereof; plant oils such as palm oil, olive oil, canola oil, camellia oil, cacao butter, shea butter, sal butter, and illipe butter; and animal fats such as fish oil, beef tallow, and lard. Among them, canola oil and a medium-chain triglyceride are preferable.

Examples of the compounds having at least one alcoholic hydroxyl group(s) in the molecular include various compounds such as monoalcohols, phytosterols, polyalcohols and amino alcohols. Specifically, there are polyalcohols such as short-chain, medium-chain, or long-chain, saturated or unsaturated, and linear or branched alcohols, glycols, glycerin, and erythritols. Among these, glycerin is preferable.

Examples of the carboxylic acids include short-chain, medium-chain, or long-chain, saturated or unsaturated, and linear or branched carboxylic acids.

Among them, there are fatty acids having 6 to 30 carbon atoms, that is, for example, octanoic acid, decanoic acid, lauric acid, myristic acid, palmitic acid, stearic acid, oleic acid, linoleic acid, and linolenic acid. They can be used singly or in combination with two kinds or more thereof. Among them, an unsaturated fatty acid is preferable, and it is particularly preferable that conjugated linoleic acid be used.

Regarding the conditions of ester exchanges, the ester changes can be conducted, for example, according to the conditions described in Japanese Unexamined Patent Publication No. Hei 13-169795. To take a single example, a medium-chain triglyceride can be obtained by ester-exchanging canola oil and a medium-chain triglyceride: ODO (The Nisshin OilliO Group, Ltd.) with a lipase for ester exchanges of the present invention.

Meanwhile, in case of using a lipase in the process (a) originated from *Penicillium* sp. and the powdery form thereof, it is better to initiate the reaction by previously adding 0.1 to 5 weight% of water to a total mass of a substrate and to conduct the reaction after that.

The lipase obtained by the production method of the present invention can be repeatedly used by being reproduced by filtration. In case of using a reproduced lipase, the reproduced lipase can be used by itself or it can be used in combination with unused lipase obtained by the present invention.

According to the first embodiment of the present invention, the purified lipase can be obtained wherein content of impurity such as silicon is decreased. Particularly, the lipase can be obtained wherein the content of silicon is decrease to 100ppm or less. Besides, the fat and fatty oil composition can be obtained wherein flavor and color are good. Further, the fat and fatty oil composition can also be obtained wherein foaming is inhibited when food is fried and, therefore, workability of cooking is high.

### Example 1

90g of canola oil, 10g of ODO (The Nisshin OilliO Group, Ltd.), and 20g of a filter aid, KC Flock (Nippon Paper Chemicals Co., Ltd.) were added to 20g of Lipase QLM produced by Meito Sangyo Co., Ltd. The mixture was stirred at 25°C for 5 hours and then filtered. 90g of canola oil and 10g of ODO (The Nisshin OilliO Group, Ltd.) were replaced with other oils and solvents to conduct the same experiment. Then, content of silicon in the lipase after the treatment was determined by HPLC (Shimazdu Corporation).

Next, 0.5% of the obtained lipase was added to150g of ODO (The Nisshin OilliO Group, Ltd.) and 850g of canola oil (The Nisshin OilliO Group, Ltd.). The mixture was stirred for 15 hours and the ester exchange reaction was conducted. After the completion of the reaction, the lipase was filtered. Then, the obtained oil was deacidified, decolored and deodorized by the known method to prepare ester-exchanged oil.

The color of the obtained ester-exchanged oil was determined by Lobbybond and the flavor of oil was compared by a trained panelist(s).

### Result

As shown in the following table, an amount of silicon could be decreased by mixing a long chain fatty acid triglyceride with a medium-chain triglyceride and pretreating the mixture.

Further, it was clarified that, when the ester exchange reaction was conducted with the purified lipase obtained by the production method of the present invention, the fat and fatty oil having good color and flavor.

**[Table 1]**

| | Silicon (ppm) | Color of ester-exchanged oil | Flavor of ester-exchanged oil |
|---|---|---|---|
| Before treatment | 5000 | 11Y5R | × |
| Canola oil /ODO=9/1* | 100 or less | 5Y2R | ○ |
| ODO only | 1800 | 8Y3R | Δ |
| Canola oil only | 2300 | 9Y4R | Δ |
| Hexane | 3000 | 7Y3R | × |
| THF | 1800 | 10Y5R | × |
| Octanoic acid | 4000 | 10Y4R | × |

| | | | |
|---|---|---|---|
| * : Example | | | |

### Example 2

90g of canola oil, 10g of ODO (The Nisshin OilliO Group, Ltd.), 100g of monoglyceride (Sunsoft 8090, produced by Taiyo Kagaku Co., Ltd.) and 20g of a filter aid, KC Flock (Nippon Paper Chemicals Co., Ltd.) were added to each 20g of various lipases. The mixture was stirred at 25°C for 5 hours and then filtered. Powder lipases RM and TL were produced by ultrafiltering commercially available enzyme solutions (Palatase and Lipozyme TL100L) and then powderizing them with a spray dryer. The ester-exchanged oils were produced from the obtained lipases as the same method as that of Example 1. The colors of the ester-exchanged oils were determined and the flavors of oils were evaluated.

**[Table 2]**

| Lipase | Silicon (ppm) | Color of ester-exchanged oil | Flavor of ester-exchanged oil |
|---|---|---|---|
| Lipase QLM (Meito) | 100 or less | 7Y3R | ○ |
| Lipase PL (Meito) | 80 or less | 6Y3R | ○ |
| Lipase RM(NOVOZYMES) | 50. or less | 8Y2R | ○ |
| Lipase TL(NOVOZYMES) | 50 or less | 8Y3R | ○ |
| Lipase D (Amano ENZYME) | 50 or less | 7Y2R | ○ |

### Example 3

90g of canola oil, 10g of ODO (The Nisehin OilliO Group, Ltd.), and 20g of a filter aid, KC Flock (Nippon Paper Chemicals Co., Ltd.) were added to 20g of Lipase PL produced by Meito Sangyo Co., Ltd. The mixture was stirred at 25°C for 5 hours and then filtered. The ester-exchanged oils were produced from the obtained lipase as the same method as that of Example 1. The colors of the ester-exchanged oils were determined and the flavors of oils were evaluated.

**[Table 3]**

| Result | | | |
|---|---|---|---|
| Silicon (ppm) | Color of ester-exchanged oil | Flavor of ester-exchanged oil | |
| 80 or less | 5Y3R | ○ | |
| | | | |

| | Silicon (ppm) | Color of ester-exchanged oil | Flavor of ester-exchanged oil |
|---|---|---|---|
| Before treatment | 3000 | 12Y6R | × |
| Canola oil /ODO=9/1 | 100! or less | 6Y3R | ○ |

### Example 4

90g of canola oil, 10g of ODO (The Nisshin OilliO Group, LtdJ, 200g of diglyceride obtained by hydrolysis and molecular-distillation of canola oil and 20g of a filter aid, KC Flock (Nippon Paper Chemicals Co., Ltd.) were added to 20g of Lipase QLM. The mixture was stirred at 25°C for 5 hours and then filtered. The ester-exchanged oil was produced from the obtained lipase as the same method as that of Example 1. The colors of the ester-exchanged oils were determined and the flavors of oils were evaluated.

**[Table 4]**

| | Silicon (ppm) | Color of ester-exchanged oil | Flavor of ester-exchanged oil |
|---|---|---|---|
| | 100 or less | 5Y3R | ○ |

### Example 5

90g of canola oil, 10g of ODO (The Nisshin OilliO Group, Ltd.), 100g of monoglyceride (Sunsoft 8090, produced by Taiyo Kagaku Co., Ltd.) and 20g of a filter aid, KC Flock (Nippon Paper Chemicals Co., Ltd.) were added to each 20g of various lipases. The mixture was stirred at 25°C for 5 hours and then filtered. Powder lipases RM and TL were produced by ultrafiltering commercially available enzyme solutions (Palatase and Lipozyme TL100L) and then powderizing them with a spray dryer.

Next, 0.5% of the obtained lipase was added to 150g of ODO (The Nisshin OilliO Group, Ltd.) and 850g of canola oil (The Nisshin OilliO Group, Ltd.). The mixture was stirred for 15 hours and the ester exchange reaction was conducted. After the completion of the reaction, the lipase was filtered. Then, the obtained oil was deacidified, decolored and deodorized by the known method to prepare ester-exchanged oil.

The ester exchange activity of the obtained lipase was determined in accordance with the disclosure of S. Negishi, S. Shirasawa, Y Arai, J. Suzuki, S. Mukataka, Enzyme Microb. Technol, 32, 66-70(2003), and shown as relative values.

**[Table 5]**

| Lipase | Activity before treatment | Activity after treatment |
|---|---|---|
| Lipase QLM (Meito Sangyo) | 1 | 3.1 |
| Lipase PL (Meito Sangyo) | 1 | 2.8 |
| Lipase RM (NOVOZYMES) | 1 | 4.1 |
| Lipase TL (NOVOZYMES) | 1 | 2.6 |

### Example 6

Total of 100g of monoglyceride (Sunsoft 8090, produced by Taiyo Kagaku Co., Ltd.) and triglyceride wherein canola oil and ODO (The Nisshin OilliO Group, Ltd.) were combined at the rate of 9/1, and 20g of a filter aid, KC Flock (Nippon Paper Chemicals Co., Ltd.) were added to 20g of Lipase QLM. The mixture was stirred at 60°C for 3 hours and then filtered. At that time, the rate of triglyceride and monoglyceride was changed. The ester-exchanged oils were produced from the obtained lipases as the same method as that of Example 6, and their ester exchange activities were determined.

**[Table 6]**

| Rate of triglyceride / monoglyceride | Activity before treatment | Activity after treatment |
|---|---|---|
| 1/0∗ | 1 | 1.8 |
| 2/1 | 1 | 2.1 |
| 1/1 | 1 | 2.5 |
| 1/2 | 1 | 3.7 |
| 1/5 | 1 | 4.0 |
| 1/10 | 1 | 3.6 |

| | | |
|---|---|---|
| * Comparative Example | | |

### Example 7

90g of canola oil, 10g of ODO (The Nisshin OilliO Group, Ltd.), 200g of diglyceride obtained by hydrolysis and molecular-distillation of canola oil and 20g of a filter aid, KC Flock (Nippon Paper Chemicals Co., Ltd.) were added to 20g of Lipase QLM. The mixture was stirred at 25°C for 5 hours and then filtered. The ester-exchanged oil was produced from the obtained lipase as the same method as that of Example 6, and its ester exchange activity was determined.

**[Table 7]**

| | Activity before treatment | Activity after treatment |
|---|---|---|
| Canola oil I/ODO/DGA=9/1/20 | 1 | 5.2 |

## Claims

1. A method for producing a purified lipase which comprises the steps of:
(a) bringing a long chain fatty acid triglyceride and a medium-chain triglyceride into contact with a lipase by stirring a mixture of said triglycerides, lipase and a filter aid for 0.5 to 100 hours at ambient temperature to purify the lipase wherein 2 to 100 times mass of triglyceride is brought into contact with a total mass of the lipase, and
(b) collecting the purified lipase by filtration.

2. The method for producing the lipase according to Claim 1, wherein the long chain fatty acid triglyceride is a triglyceride of which constituent fatty acid has 14 to 24 carbon atoms and the medium-chain triglyceride is a triglyceride of which constituent fatty acid has 6 to 12 carbon atoms.

3. The method for producing the lipase according to Claim 2, wherein the long chain fatty acid triglyceride is selected from the group consisting of canola oil, soybean oil, sunflower oil, safflower oil and corn oil.

4. The method for producing the lipase according to any one of Claims 1 to 3, wherein the long chain fatty acid triglyceride and the medium-chain triglyceride are used in the mass ratio of 95:5 to 50:50.

5. The method for producing the lipase according to any one of Claims 1 to 4, wherein the lipase is a lipase originated from Alcaligenes sp., Pseudomonas sp., Rhizomucor sp., Mucor sp., Thermomyces sp., Rhizopus sp., or Penicillium sp.

6. The method for producing the lipase according to any one of Claims 1 to 5, wherein the lipase is a powder lipase, and 90 wt% or more of said powder lipase are particles having a size of 1 to 100µm.

7. The method for producing the lipase according to any one of Claims 1 to 6, wherein the water content of the lipase is adjusted to 0.1 to 10% before or after the reaction.

## Patentansprüche

1. Verfahren zur Herstellung einer gereinigten Lipase umfassend die Schritte:
(a) Bringen eines langkettigen Fettsäuretriglycerids und eines mittel-kettigen Triglycerids in Kontakt mit einer Lipase durch Verrühren einer Mischung der besagten Triglyceride, Lipase und eines Filterhilfsmittels für 0,5 bis 100 Stunden bei Raumtemperatur zur Reinigung der Lipase, wobei eine 2- bis 100-fache Masse des Triglycerids in Kontakt mit einer Gesamtmasse der Lipase gebracht wird, und
(b) Sammeln der gereinigten Lipase durch Filtration.

2. Verfahren zur Herstellung einer gereinigten Lipase gemäß Anspruch 1, wobei das langkettige Fettsäuretriglycerid ein Triglycerid ist, dessen Fettsäurebestandteil 14 bis 24 Kohlenstoffatome hat und das mittel-kettige Triglycerid ein Triglycerid ist, dessen Fettsäurebestandteil 6 bis 12 Kohlenstoffatome hat.

3. Verfahren zur Herstellung einer gereinigten Lipase gemäß Anspruch 2, wobei das langkettige Triglycerid ausgewählt ist aus der Gruppe bestehend aus Raps-Öl, Soyabohnen-Öl, Sonneblumen-Öl, Färberdistel-Öl, und Mais-Öl.

4. Verfahren zur Herstellung einer gereinigten Lipase gemäß einem der Ansprüche 1 bis 3, wobei das langkettige Fettsäuretriglycerid und das mittel-kettige Triglycerid im Massenverhältnis von 95:5 bis 50:50 verwendet werden.

5. Verfahren zur Herstellung einer gereinigten Lipase gemäß einem der Ansprüche 1 bis 4, wobei die Lipase eine Lipase ist, die von *Alcaligenes sp., Pseudomonas sp., Rhizomucor sp., Mucor sp., Thermomyces sp., Rhizopus sp.,* oder *Penicillium sp.* Stammt.

6. Verfahren zur Herstellung einer gereinigten Lipase gemäß einem der Ansprüche 1 bis 5,wobei die Lipase eine Pulver Lipase ist, und 90 Gewichts-% oder mehr der besagten Pulver Lipase Partikel sind, die eine Größe von 1 bis 100 µm haben.

7. Verfahren zur Herstellung einer gereinigren Lipase gemäß einem der Ansprüche 1 bis 6, wobei der Wassergehalt der Lipase auf 0,1 bis 10% vor oder nach der Reaktion eingestellt ist.

## Revendications

1. Procédé de production d'une lipase purifié qui comprend les étapes consistant à :
(a) mettre en contact un triglycéride d'acide gras à chaîne longue et un triglycéride à chaîne moyenne avec une lipase en agitant un mélange constitué desdits triglycérides, de ladite lipase et d'un agent d'aide à la filtration pendant 0, 5 à 100 heures à température ambiante pour purifier la lipase, dans lequel une proportion de 2 à 100 fois la masse de triglycéride est mise en contact avec une masse totale de la lipase, et
(b) collecter la lipase purifiée par filtration.

2. Procédé de production de la lipase selon la revendication 1, dans lequel le triglycéride d'acide gras à chaîne longue est un triglycéride dont l'acide gras constitutif présente 14 à 24 atomes de carbone et le triglycéride à chaîne moyenne est un triglycéride dont l'acide gras constitutif présente 6 à 12 atomes de carbone.

3. Procédé de production de la lipase selon la revendication 2, dans lequel le triglycéride d'acide gras à chaîne longue est sélectionné dans le groupe constitué de l'huile de colza, de l'huile de fèves de soja, de l'huile de tournesol, de l'huile de carthame et de l'huile de maïs.

4. Procédé de production de la lipase selon l'une quelconque des revendications 1 à 3, dans lequel le triglycéride d'acide gras à chaîne longue et le triglycéride à chaîne moyenne sont utilisés dans un rapport massique de 95:5 à 50:50.

5. Procédé de production de la lipase selon l'une quelconque des revendications 1 à 4, dans lequel la lipase est une lipase provenant des espèces Alcaligenes sp., Pseudomonas sp., Rhizomucor sp., Mucor sp., Thermomyces sp., Rhizopus sp. ou Penicillium sp.

6. Procédé de production de la lipase selon l'une quelconque des revendications 1 à 5, dans lequel la lipase est une lipase en poudre, et 90 % en poids ou plus de ladite lipase en poudre sont des particules ayant une taille de 1 à 100 µm.

7. Procédé de production de la lipase selon l'une quelconque des revendications 1 à 6, dans lequel la teneur en eau de la lipase est ajustée à 0,1 à 10 % avant ou après la réaction.
